# EUROPEAN PATENT APPLICATION

(11) **EP 4 356 817 A1**
(43) Date of publication of application: **24.04.2024**
(21) Application number: 22202621.3
(22) Date of filing: 19.10.2022
(51) Int. Cl.: A61B 3/00

(54) **AN OPHTHALMOLOGICAL MEASUREMENT SYSTEM COMPRISNG AN OPHTHALMOLOGICAL MEASURMENT DEVICE AND A DEVICE FOR VERIFICATION OF FUNCTIONALITY AND/OR A STATE OF CALIBRATION OF THE OPHTHALMOLOGICAL MEASUREMENT DEVICE COMPRISING A MAGNETIC ELEMENT**

(71) Applicant: Carl Zeiss Vision International GmbH, 73430 Aalen (DE)
(72) Inventor: Schneider, Martina, 86720 Nördlingen (DE); Buehren, Tobias, 89073 Ulm (DE); Berghoff, Patrick, 73434 Aalen (DE)
(74) Representative: Carl Zeiss AG - Patentabteilung

(57) **Abstract**

This application relates to a device for verification of a state of calibration and/or functionality of an ophthalmological measurement device (306) for biometric data of an eye which provides a magnetic attachment. The application further relates to an ophthalmological measurement system (300), comprising an ophthalmological measurement device (306) for biometric data of an eye and a device for verification of a state of calibration and/or functionality (302), wherein the device for verification (302) is configured to be mountable on the ophthalmological measurement device (306) via magnetic force.

## Description

### FIELD OF THE INVENTION

The present invention relates to an ophthalmological measurement system for measurement and analysis of biometric data of an eye of a user, which requires a device for verification of functionality and/or a state of calibration of the ophthalmological measurement system. The present invention also relates to the device for verification of functionality and/or a state of calibration of the system.

### BACKGROUND OF THE INVENTION

For ophthalmological measurement system, a recurring verification of a state of calibration and/or functionality, and potential subsequent adjustment is necessary during the routine process to ensure the desired measurement quality, accuracy, and proper functioning of the system.

In this regard, it is required to regularly attach a device for verification of functionality and/or a state of calibration of ophthalmological measurement system, or shortly a device for verification, such as a model-eye or a test eye with a predetermined reference value to the ophthalmological measurement system, measure, and then compare the actual measured value of said device with its reference value to determine the correction value. Said process also verifies the functionality of the system to guarantee the accurate performance of the measurement system.

This applies particularly to systems for biometry, keratometry, topography, and/or auto-refraction of the eye, where complex light structures, such as slices, rings, slits, or the like, are projected, sometimes simultaneously, with large and/or very different angles onto the eye. The most important parameters to be determined are, for example, among others, axial length, corneal curvature, corneal astigmatism, corneal diameter or refractive power as well as the length of the anterior chamber (distance to the eye lens).

According to the prior art, ophthalmological measurement system of the biometric data of an eye as well as a device for verification of a state of calibration and/or functionality of the system are sufficiently known. Various types of device for verification of a state of calibration and/or functionality of the system are well-known and have been used throughout the ophthalmic industry, some very simple with poor imaging quality, others are more complicated, imitating the structure of the human eye (cornea and lens) and achieving high optical quality at great cost. A common version of a device for verification of a state of calibration and/or functionality is a solid glass or plastic component with a curved front surface and a flat back surface. The front curve serves the role of a cornea for the calibration device, and the back surface serves as a retina for the device for verification. Some devices for verification have a limiting aperture that serves as an iris. The aperture is most commonly located in front of the front surface of the device for verification, but it can also be inside the device for verification.

The term "calibration" refers to set of operations which establishes, by reference to standards, the relationship which exists, under specified conditions, between an indication and a result of a measurement. To be specific, in an ophthalmological measurement system, calibration indicates a process of comparing the accuracy of a measurement of ophthalmological measurement system to a known standard and then adjusting the system's accuracy to deliver reliable measurements based on the standard. The term "verification of a state of calibration " indicates set of operations which is used to check whether the indications, under specified conditions, correspond with a given set of known measurements within the limits of a predetermined calibration diagram. That is, verification of a state of calibration in an ophthalmological measurement system is a procedure periodically ensuring that a system is working as intended and that any error is within the tolerance (maximum permissible error) (International Electrotechnical Commission Electropedia under https://www.electropedia.org/).

The term "functionality" refers to the range of operations that can be run on a system. In the present invention, the "functionality of the (ophthalmological measurement) system" means that the operations conducted by the ophthalmological measurement system.

In the conventional ophthalmological measurement systems, the device for verification of a state of calibration and/or functionality such as a test eye or a model eye is usually designed as a separate accessory and utilized at regular intervals for testing the functionality and the state of calibration of the system. Since it is provided as a separate, individual unit of the system, the device for verification of a state of calibration and/or functionality has to be manually attached to the system upon use.

For this, most of the conventional ophthalmological measurement systems come with a device for verification of a state of calibration and/or functionality which can be fixed on the system with screws, bolts or studs. For instance, Huvitz HRK 9000A (autorefractor/keratometer) uses pin on a chin rest of the system and the holes in the model eye to fix a device for verification, a model eye, to the system. Oculus/Nidek ARK-1 uses studs to fix a device for verification of a state of calibration and/or functionality, a test eye, to the system. Zeiss Meditec IOLMaster 500 fixes a test eye with screws into the holes of a chin rest of the system. US 2007/0291277 A1 uses a conventional test eye which has to be attached manually. Such installations require considerable effort to manually fix a test eye on the device each and every time of the installation. Further, a user must exert force to manually align position and orientation of a test eye, which would lead to rather imprecise alignment of the device for verification.

In addition, as a device for verification of a state of calibration and/or functionality is provided as a separate accessory, ophthalmological measurement systems typically have no dedicated storage space for the device for verification. This causes the device for verification to get frequently lost and be prone to damage and contamination.

### SUMMARY OF THE INVENTION

The conventional ophthalmological measurement systems are disadvantageous because use of an external device for verification of a state of calibration and/or functionality of the system such as a test eye is laborious for the user. This disadvantage arises especially from the installation, alignment and storage of the device for verification. To overcome said drawbacks of the conventional ophthalmological measurement system using an external device for verification, the present invention provides a novel ophthalmological measurement system comprising a novel device for verification of the system, namely, a device for verification of a state of calibration and/or functionality such as a test eye or a model eye.

According to the present invention, the task is solved by providing a device for verification of a state of calibration and/or functionality of an ophthalmological measurement system, which is attached to the system via magnetic force. Instead of the conventional means for connection such as screws, bolts, or studs, which require considerable amount of time and effort for manual installation and result in inaccurate alignment, the present invention utilizes magnetic force between an ophthalmological measurement device and a device for verification of a state of calibration and/or functionality, thereby significantly reducing the effort and time required for the installation.

Additionally, by providing a separate storage space in the system, the present invention provides easy maintenance of the device for verification of a state of calibration and/or functionality.

### BRIEF DESCRIPTION OF THE DRAWINGS

In the following, the solution of the present invention is further described by means of embodiment examples.
FIG 1A and 1B show a conventional device for verification of a state of calibration and/or functionality of a conventional ophthalmological measurement system using studs on a chin rest for the connection (Oculus/Nidek ARK-1).
FIG 2A, 2B and 2C show one embodiment of a device for verification of a state of calibration and/or functionality of the present invention configured to be mountable on a head rest of the ophthalmological measurement device via magnetic force.
FIG 3 illustrates another example of a device for verification of a state of calibration and/or functionality of the present invention configured to be mountable on a head rest of an ophthalmological measurement device via magnetic force.
FIG 4 illustrates an embodiment of a storage unit in an ophthalmological measurement device for a device for verification of a state of calibration and/or functionality.

### DETAILED DESCRIPTION OF THE INVENTION

The present invention relates to an ophthalmological measurement system, for example, for biometry, keratometry, topography or auto-refractor of the eye where a device for verification of a state of calibration and/or functionality of the ophthalmological measurement system is provided as an individual, separate unit. The present invention aims to provide an ophthalmological measurement system and/or a device for verification of a state of calibration and/or functionality which enables convenient and user-friendly installation of the device for verification of a state of calibration and/or functionality at the system. The present invention further relates to the device for verification which can be easily mounted on and oriented on an ophthalmological measurement device of the system.

In the first aspect of the present invention, an ophthalmological measurement system comprising (i) an ophthalmological measurement device for biometric data of an eye and (ii) a device for verification of a state of calibration and/or functionality which verifies a state of calibration and/or functionality of the ophthalmological measurement device, characterized in that the device fori verification is configured to be mountable on the ophthalmological measurement device via magnetic force is provided.

The term "ophthalmological measurement device" refers to a unit of the ophthalmological measurement system which measures biometric data of an eye. The term a "device for verification of a state of calibration and/or functionality" or a "device for verification" indicates a device which verifies a state of calibration and/or functionality of an ophthalmological measurement device or system.

The system of the present invention provides the device for verification of a state of calibration and/or functionality comprising a first magnetic surface and the ophthalmological measurement device comprising a second magnetic surface. With the magnetic surfaces, the device for verification can be mounted on the ophthalmological measurement device and the device for calibration via magnetic force and the ophthalmological measurement device are coupled via magnetic force and provide a magnetic attachment.

The ophthalmological measurement system for biometric data of an eye includes autorefractors, keratometers, aberrometers, fundus cameras, corneal topographers, OCT-based imaging systems, wavefront sensors, and/or laser eye surgery systems, etc.

The device for verification evaluates or verifies a state of calibration of an ophthalmological measurement done by the system and/or functionality of the system. The device for verification can be in a form of test eyes or model eyes that are commonly used in the relevant technical field. Preferably, the device for verification is not limited thereto but typically comprises at least one curved surface as test structure. Hereby, possible test structures are test surfaces, particularly test spheres, for the determination of the radius of curvature on the cornea and/or eye lens; spacing structures for the calibration of measurements of partial sections such as axis length or anterior chamber depth; reflectors with determined degrees of reflection (attenuators) for sensitivity determination; and/or test patterns for resolution tests and lateral calibration of fundus or anterior chamber images. It is understood that any devices for verification such as test eyes or model eyes that are commercially available and/or commonly used can be selected and used as needed depending on the ophthalmological measurement device to be verified.

The goal of the present invention can be achieved by magnetic elements. In this regard, the system of the present invention is further characterized in that the device for verification of a state of calibration and/or functionality comprises a first magnetic element that is coupled to a second magnetic element of the ophthalmological measurement device. In this case, the first magnetic element and the second magnetic element form the first magnetic surface and the second magnetic surface. In other words, a surface comprising a magnetic element becomes a magnetic surface. The coupled magnetic elements generate magnetic force and tract each other, thereby forming a magnetic attachment. The magnetic elements allow mounting of the device for verification on the measurement device and/or a removable and releasable coupling of the device for verification and the ophthalmological measurement device via magnetic force.

The term "magnetic element" refers to magnets, which are also referred to as permanent magnet and produce their own persistent magnetic field even in the absence of an applied magnetic field, as well as magnetic materials, which are also referred to as temporary magnet and can produce a magnetic field in response to an applied magnetic field (magnetism), and thus become magnetized in the presence of a magnetic field. Magnetic elements can also be any materials or combinations of materials that are magnetically attracted to one another and generate magnetic field and force. Examples of magnet are neodymium iron boron (NdFeB or NIB), samarium cobalt (SmCo), ferrite (ceramic magnets, e.g., BaFe₂O₃ or SRFe₂O₃) and alnico magnet (Al-Ni-Co). Magnetic materials may be, for example, a ferromagnetic material or ferrimagnetic materials such as irons, iron alloys, or steels.

The magnetic elements produce a magnetic force to retain the device for verification to be fixed to the ophthalmological measurement device. The magnetic force refers to magnetic traction between the two devices which provides magnetic attachment.

The first magnetic element of the device for verification and the second magnetic element of the ophthalmological measurement device can be a pair of magnets which are attracted to each other, or a magnet and a magnetic material. Preferably, at least one of the first and the second magnetic elements can be magnets. More preferably, both of the first and the second magnetic elements can be magnets. It is further understood that any materials or combinations of materials that are magnetically attracted to one another and produce a magnetic attractive force can be used to achieve the magnetic force required by the present invention.

The magnetic elements can be provided on the exterior of the devices or may be hidden below the exterior material of the devices to generate a magnetic surface. In other words, the device for verification may comprise the first magnetic element on/in the first magnetic surface and the ophthalmological measurement device may comprise the second magnetic element on/in the second magnetic surface. The number of magnetic elements and/or the magnetic force formed by the magnetic elements will be controlled so that magnetic force between the devices remains strong enough to form a reliable attachment. The magnetic force can readily be selected and applied as necessary by a person skilled in the art to achieve a stable fixation as well as an easy installation and deinstallation, depending on shape, material, weight direction of the devices.

Conventionally, test eyes or model eyes are manually installed at a chin rest of an ophthalmological measurement device. According to the present invention, the second magnetic element of the ophthalmological measurement device is placed in/on/at a head rest and/or a chinrest so that the first magnetic element of the device for verification can be mounted on the head rest and/or the chinrest of the ophthalmological measurement device via magnetic force.

The term "chin rest" is a part attached to an ophthalmological measurement device to aid positioning of a subject's chin, ultimately the head, during operation of the device. The chin rest usually has a recess in a shape of a chin (chin cup) so that the subject's chin can be put, and the head can be firmly and comfortably held. The term "head rest" or "forehead rest" is a part of an ophthalmological measurement device to aid positioning of a subject's forehead, ultimately the head, during operation of the device. The head rest usually has a recess in a shape of a forehead so that the subject's forehead can be put, and the head can be comfortably held.

In a preferred embodiment, the device for verification of a state of calibration and/or functionality is arranged directly at the head rest of the ophthalmological measurement device. Unlike the conventional ophthalmological measurement devices, the present invention mounts the device for verification on the head rest thereby resulting in a more simplified alignment of the position and orientation of the device for verification. This is because in an ophthalmological measurement device, a chin rest and/or a motorized supporting part of a chin rest is generally designed to be movable and adjustable in accordance with a patient's anatomical features such as position of the eyes, face length, etc. so that the patient's eyes can be centered at an appropriate position during the measurement. Therefore, for a centration of a device for verification attached to the chinrest, two variables with different movement ranges of the chinrest as well as the measurement device needs to be considered, making the alignment more complicated. On the other hand, headrest of ophthalmological measurement device is mostly fixed and not movable. This means that once the device for verification is attached to a headrest, only the measurement device needs to be aligned to the fixed position of the device for verification. As only one variable needs to be considered, the alignment process becomes faster and less complicated. In this regard, in a preferred embodiment of the present invention, a head rest is fixed on the ophthalmological measurement device. This means that the head rest cannot be adjusted and/or moved.

Using magnetic force between the device for verification and the ophthalmological measurement device is advantageous as it is more convenient for a user to install, and still establishes reliable but releasable attachment of the device for verification simply by bringing the device for verification to a proper proximity with the second magnetic element of the ophthalmological measurement device.

Magnetic elements can be further configured to facilitate precise alignment and retention of the device for verification on the ophthalmological measurement device during verification of a state of calibration and/or functionality of the ophthalmological measurement system. During alignment through magnetic attachment, the device for verification is configured to be aligned with an accurate centration towards the ophthalmological measurement device so that the device for verification is in the axis of the measurement.

For instance, the second magnetic element of the ophthalmological measurement device may be configured so that when the first magnetic surface comprising the first magnetic element of the device for verification of a state of calibration and/or functionality is brought into a close proximity to the ophthalmological measurement device by a user, the second magnetic element pulls the device for verification towards the ophthalmological measurement device into a predetermined orientation and position that ensure precise verification of a state of calibration and/or functionality via accurate centration and prevents the device for verification from slipping off from the ophthalmological measurement device.

In another embodiment, the first and second magnetic elements are configured so that magnetic force between the device for verification of a state of calibration and/or functionality and the ophthalmological measurement device takes place only when the device for verification is oriented within a predetermined distance, position and orientation relative to the ophthalmological measurement device that ensures accurate centration. In a further embodiment, misalignments of more than a threshold amount (e.g., 10 degrees, 20 degrees) result in the device for verification not attaching to the ophthalmological measurement device, being repelled therefrom, or being attracted towards the predetermined orientation or position.

In a further embodiment, the first magnetic surface of the device for verification of a state of calibration and/or functionality and the second magnetic surface of the measurement device are configured to be interlocked with each other. In other words, the first magnetic surface is in a shape which conforms to the shape of the second magnetic surface, thereby enabling a precise engagement of the device for verification and the ophthalmological measurement device.

In an additional embodiment, at least one from the group consisting of the device for verification of a state of calibration and/or functionality, the head rest and the chin rest of the ophthalmological measurement device may comprise means for indicating a position of the device for verification to be aligned on the ophthalmological measurement device. For instance, the means can be a notch, a mark, etc.

After the initial alignment of the device for verification of a state of calibration and/or functionality via magnetic force, a fine alignment between the device for verification and the ophthalmological measurement device might be required, for example, so that the measuring beam, originating from an illumination unit and after reflection or backscattering at the device for verification, sufficiently impinges on the measuring module. The fine alignment can be done by motorized movement (e.g., remote control) and/or manual adjustment (e.g., joystick, buttons) of the devices.

In the second aspect of the present invention, a storage unit for a device for verification of a state of calibration and/or functionality is provided in the system, preferably in an ophthalmological measurement device. With a separate storage unit in the system, the device for verification can be maintained to satisfy the respective maintenance standards so that it is ensured that the device is neither contaminated nor damaged.

The storage unit may comprise a recess having a shape which conforms to a shape of a device for verification. With this feature, the device for verification can be inserted into the recess of the storage unit.

In a preferred embodiment, the storage unit and the device for verification of a state of calibration and/or functionality are coupled via magnetic force. A third magnetic surface of the storage unit and the first magnetic surface of the device for verification are coupled via magnetic force. The third magnetic surface of the storage unit forms the third magnetic surface. The third magnetic element of the storage unit is magnetically coupled to the first magnetic element of the device for verification. This enables a fixed storage of the device for verification during its non-use. In a more preferred embodiment, the third magnetic element is in the surface of the recess so that the magnetic force between the storage unit and the device for verification is generated upon insertion of the device for verification. In a preferred embodiment, at least one of the first magnetic element and the third magnetic element is a magnet.

The storage unit may further include a cover lid for the protection against damage and means for cleaning. In a further embodiment, the cover lid may be a tiltable cover lid.

In a third aspect of the present invention, a device for verification of a state of calibration and/or functionality of an ophthalmological measurement system of biometric data of an eye characterized by comprising a (first) magnetic element for a magnetic attachment is provided. In a preferred embodiment, the (first) magnetic element is a magnet. In addition, the device for verification can be configured to be mountable on a chin rest and/or a head rest of an ophthalmological measurement device, preferably on a head rest. In a preferred embodiment, the (first) magnetic element is a magnet.

In a fourth aspect of the present invention, an ophthalmological measurement device characterized by comprising a (second) magnetic element for a magnetic attachment is provided. In a preferred embodiment, the (second) magnetic element is a magnet. Further, a chin rest and/or a head rest, preferably the head rest, of the ophthalmological measurement device may comprise the (second) magnetic element. In a preferred embodiment, the (second) magnetic element is a magnet.

In the third and fourth aspects of the present invention, the magnetic attachment is an attachment to another device via a magnetic force. All the features and descriptions regarding the device for verification of a state of calibration and/or functionality of an ophthalmological measurement system provided with regard to the first aspect of the present invention can be applied to the third and fourth aspects of the present invention.

These and other embodiments are discussed below with reference to the figures, however, those skilled in the art will readily appreciate that the detailed description given herein with respect to these figures is for explanatory purposes only and should not be construed as limiting.

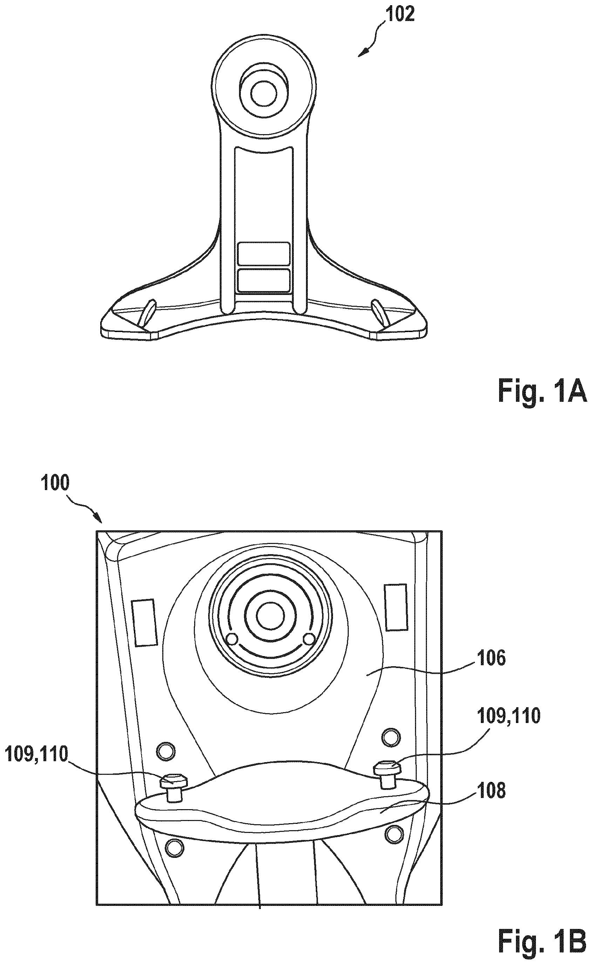

Fig. 1A and 1B illustrate a conventional device for verification of a state of calibration and/or functionality 102 of a conventional ophthalmological measurement system 100. Studs 109, 110 on a chin rest 108 of the measurement device 104 and the holes (not shown) in the device for verification 102 are fixed together and enable a manual fixation of the device for verification 102.

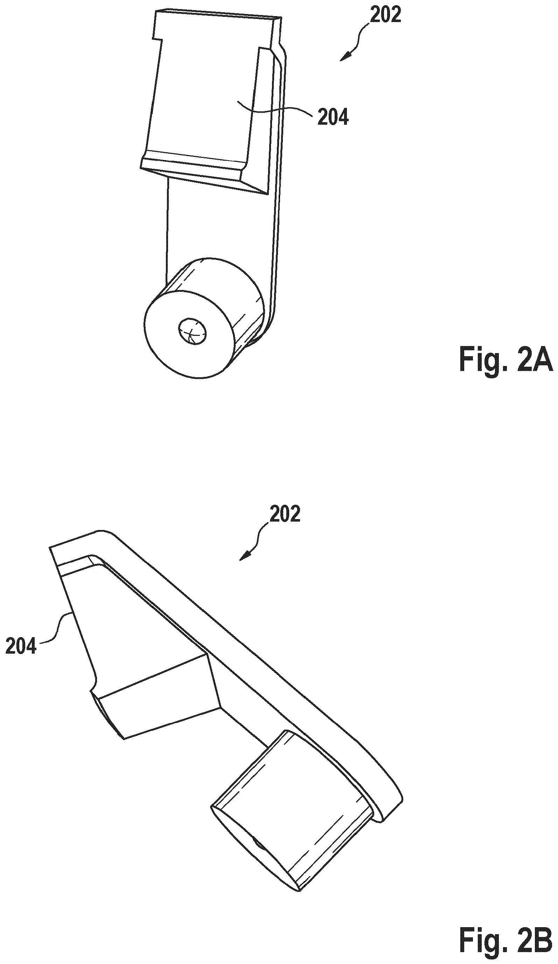

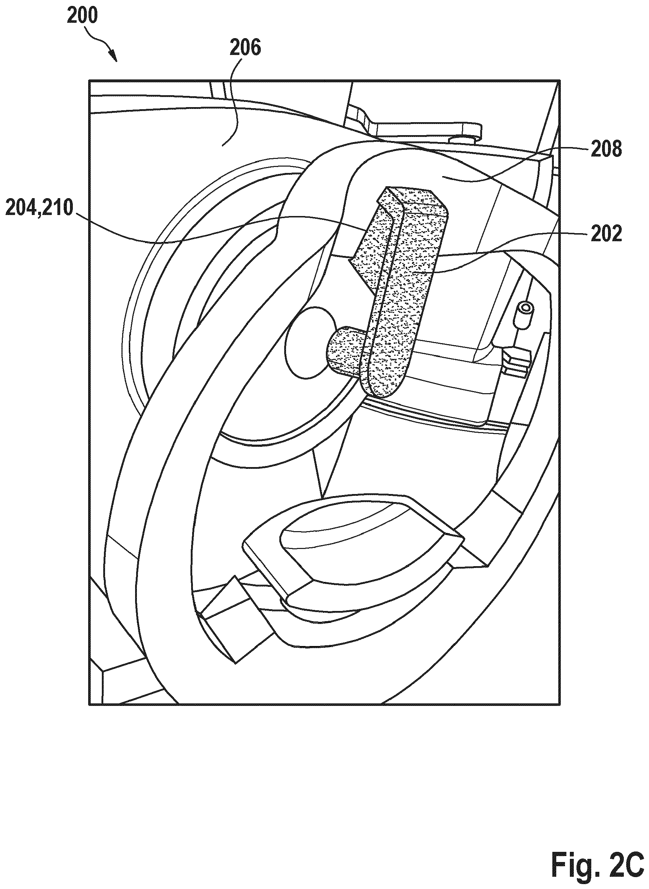

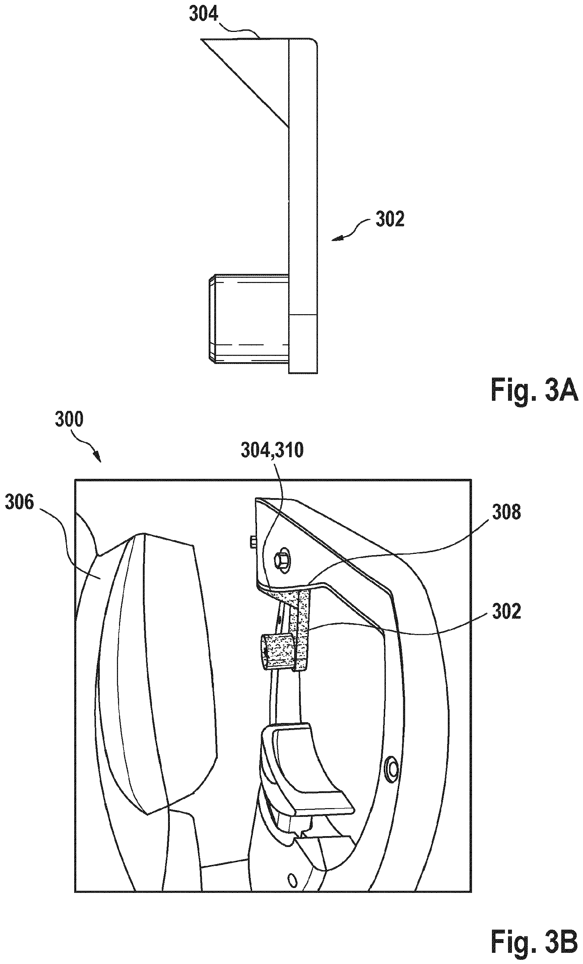

Fig. 2A shows a front view of one embodiment of a device for verification of a state of calibration and/or functionality 202 according to the present invention configured to be mountable on a head rest 208 of the ophthalmological measurement device 206 via magnetic force. A first magnetic surface and/or a first magnetic element 204 of the device for verification 202 enables the mounting of the device for verification 202 on a second magnetic surface and/or a second magnetic element 210 of the measurement device 206. Fig. 2B shows a side view of the device for verification 202. As shown in Fig. 3, an upper part of the device for verification 202 is configured to comprise a first magnetic surface and/or a first magnetic element 206 corresponding to a second magnetic surface and/or a second magnetic element 210 of a headrest 208 of the measurement device 204. These two surfaces are attached via magnetic force. The first magnetic surface 204 of the device for verification 202 and the corresponding second magnetic surface 210 of the head rest 208 comprise a magnetic element, which is in a preferred embodiment, a magnet.

FIG 3A and 3B illustrate another example of a device for verification of a state of calibration and/or functionality 302 according to the present invention configured to be mounted on a head rest 308 of an ophthalmological measurement device 306 by magnetic force. In this embodiment, the upper surface 304 of the device for verification 302 is configured to be attached to the head rest 308 of the measurement device 306 via magnetic force. Here, unlike the embodiment in FIG 2A to 2C, the top surface 304 is flat to match the shape of the head rest 308 of the measurement device 306. The bottom surface, which is a first magnetic surface and/or a first magnetic element 304, of the device for verification 302 and a second magnetic surface and/or a second magnetic element 310 of the head rest 308 of the measurement device 306 comprise magnetic elements, which, in a preferred embodiment, be a magnet. Here, the head rest 308 of the measurement device 306 is fixed and cannot be adjusted.

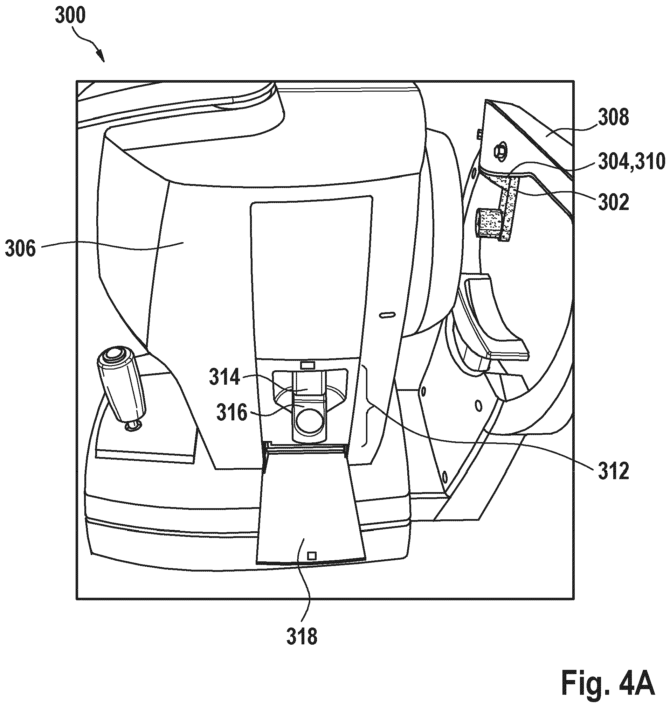

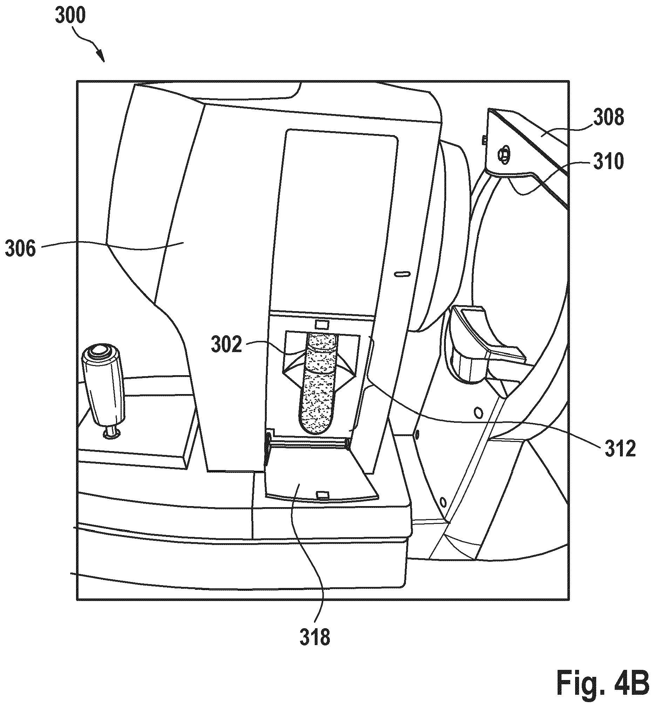

FIG 4A and 4B illustrate an embodiment of an ophthalmological measurement system 300 comprising an ophthalmological measurement device 306 and a device for verification 302, wherein the ophthalmological measurement device 306 comprises a storage unit 312. The storage unit 312 comprises a recess 314 which is configured to put in the device for verification 302. The recess 314 is configured to be shaped to match with the device for verification 302. The device for verification 302 is configured to be coupled to the storage unit 312 via magnetic force. The storage unit 312, in particular the surface of the recess 314 of the storage unit, may comprise a third magnetic element 316 that is magnetically coupled to the magnetic element of the device for verification 302 for a stronger fixation during storage. As can be seen from Fig. 4B, the storage unit may further include a tiltable cover lid 318 for the protection against damage.

## Claims

1. An ophthalmological measurement system (300), comprising an ophthalmological measurement device (306) of biometric data of an eye and a device for verification of a state of calibration and/or functionality (302) of the ophthalmological measurement device (306),
**characterized in that** the device for verification (302) is configured to be mountable on the ophthalmological measurement device 306 via magnetic force.

2. The ophthalmological measurement system (300) according to Claim 1, **characterized in that** the device for verification (302) comprises a first magnetic element (304) that is coupled to a second magnetic element (310) of the ophthalmological measurement device (306).

3. The ophthalmological measurement system (300) according to Claim 1 or 2, **characterized in that** the device (302) for verification is mountable on at least one selected from a head rest and a chin rest (308) of the ophthalmological measurement device (306) via magnetic force.

4. The ophthalmological measurement system (300) according to any one of Claims 1 to 3,
**characterized in that** the device for verification (302) is mountable on a head rest (308) of the ophthalmological measurement device (306) via magnetic force.

5. The ophthalmological measurement system according to Claim 3 or 4,
**characterized in that** the head rest (308) is fixed on the ophthalmological measurement device (306).

6. The ophthalmological measurement system (300) according to any one of Claims 2 to 5,
**characterized in that** a head rest and/or a chin rest (308) of the ophthalmological measurement device (306) comprises the second magnetic element (310).

7. The ophthalmological measurement system (300) according to any one of Claims 2 to 6,
**characterized in that** the first magnetic element (304) and/or the second magnetic element (310) comprise a magnet.

8. The ophthalmological measurement system (300) according to any one of Claims 1 to 7,
**characterized in that** the ophthalmological measurement device (306) further comprises a storage unit (312) of the device for verification (302).

9. The ophthalmological measurement system (300) according to Claim 8,
**characterized in that** the storage unit (312) comprises a recess (314) having a shape which conforms to a shape of the device for verification (302), wherein the device for verification (302) is coupled to the recess (314) via magnetic force.

10. The ophthalmological measurement system according to Claim 8 or 9,
**characterized in that** the storage unit (312) comprises a third magnetic element (316) that is magnetically coupled to the first magnetic element (304) of the device for verification (302).

11. A device for verification of a state of calibration and/or functionality (302) of an ophthalmological measurement device (306) for biometric data of an eye,
**characterized by** comprising a magnetic element (304) for magnetic attachment.

12. The device (302) according to Claim 11,
**characterized in that** the magnetic element (304) is a magnet.

13. The device (302) according to Claim 11 or 12,
**characterized in that** the device is configured to be mountable on the headrest of an ophthalmological measurement device (306).

14. An ophthalmological measurement device (306) for biometric data of an eye, **characterized by** comprising a magnetic element (310) for magnetic attachment.

15. The device (306) according to Claim 14,
**characterized in that** the magnetic element (310) is a magnet.
